# FASCICULE DE BREVET EUROPEEN

(11) **EP 0 867 436 B1**
(45) Date de publication et mention de la délivrance du brevet: **09.06.2004**
(21) Numéro de dépôt: 98400711.2
(22) Date de dépôt: 26.03.1998
(51) Int. Cl.: C07D 405/04, C07D 409/04, C07D 411/04, C07D 471/04, A61K 31/445, A61K 31/40

(54) **Dérivés de N-aryl pipéridine et leur utilisation comme ligands du récepteur 5-HT1B**
N-Aryl-piperidinderivate und ihre Verwendung als 5-HT1B-Rezeptorliganden
N-aryl-piperidine derivatives and their use as 5-HT1B receptor ligands

(30) Priorité: 27.03.1997 FR 9703760
(43) Date de publication de la demande: 30.09.1998
(73) Titulaire: LES LABORATOIRES SERVIER, 92200 Neuilly sur Seine (FR)
(72) Inventeur: Peglion, Jean-Louis, 78110 Le Vesinet (FR); Goument, Bertrand, 78220 Viroflay (FR); Millan, Mark, 78230 Le Pecq (FR); Gobert, Alain, 93200 Saint Denis (FR); Audinot, Valérie, 78300 Poissy (FR)

(56) Documents cités:
- EP-A- 0 745 598
- WO-A-95/07274
- US-A- 5 464 834

## Description

La présente invention a pour objet de nouveaux composés de N-aryl pipéridine, leur procédé de préparation et les compositions pharmaceutiques qui les contiennent.

Elle concerne plus spécifiquement :
les composés de N-aryl pipéridine de formule I : 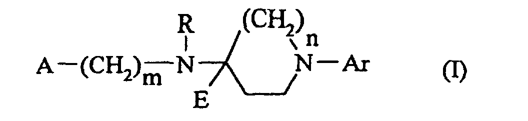
dans laquelle:
. m représente un nombre entier de 1 à 5 inclus ;
. n représente 2 ;
. R représente un atome d'hydrogène ou un radical aralkyle dans lequel la partie alkyle renferme de 1 à 5 atomes de carbone en chaine droite ou ramifiée ;
. E représente un atome d'hydrogène;
. Ar représente : 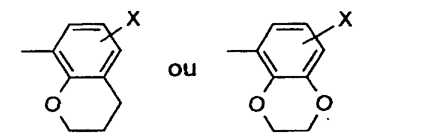 X étant un atome d'hydrogène
   ou d'halogène
. et A représente : 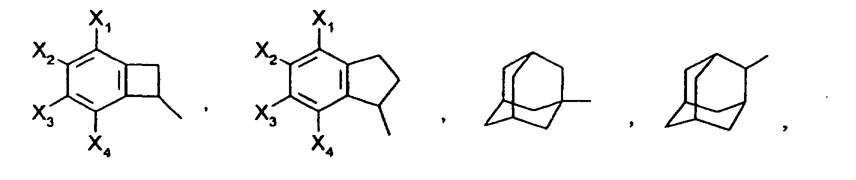 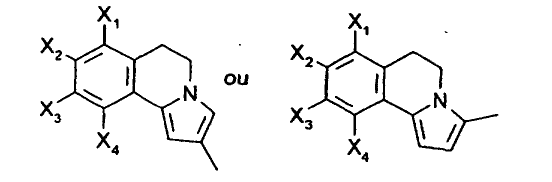
dans lesquels :
. X₁, X₂, X₃ et X₄, identiques ou différents représentent
chacun un atome d'hydrogène ou d'halogène ou un radical (C₁-C₅)alkoxy en chaîne droite ou ramifiée,
sous forme de mélange racémique et d'isomères optiques quand ils existent,
et leurs sels d'addition acides physiologiquement tolérables.

Les produits de la présente invention peuvent être utilisés comme médicaments dans le traitement de maladies dans lesquelles a été démontré l'implication du système sérotoninergique telles que les maladies psychiatriques (dépression, anxiété, attaque de panique, schizophrénie, agressivité, troubles impulsifs, troubles obsessionnels compulsifs), les maladies dégénératives (Parkinson, Alzheimer), la douleur, la migraine, les céphalées, les accidents vasculaires cérébraux, la boulimie, l'anorexie, l'abus de drogue et aussi dans les maladies cardiovasculaires (angor instable), puisqu'à l'instar du système nerveux central, le système sérotoninergique est aussi présent dans les territoires cardiovasculaires.
De nombreux récepteurs à la sérotonine ont été identifiés et récemment clonés. Ils ont été classés, sur la base de leur structure primaire et de leur mode de couplage avec les systèmes de transduction, en sept classes majeures 5-HT₁ à 5-HT₇ (cf. F.G. Boess, Molecular Biology of 5-HT Receptors, *Neuropharmacol*., **1994**, 33, 275). Ces classes sont elles-mêmes subdivisées en sous-types. Pour le récepteur 5-HT₁ on connaît les sous-types 5-HT_{1A}, 5-HT_{1B} (anciennement 5-HT_{1Dβ}) et 5-HT_{1D} (anciennement 5-HT_{1Dα}) (pour une revue récente et une discussion de la nomenclature, voir R.P. Hartig, *Trends in Pharmacol*. *Sciences,* **1996,** 17, 103).

L'application de la présente invention concerne plus particulièrement le récepteur 5-HT_{1B} et les produits revendiqués se comportent comme des ligands puissants et sélectifs de ce récepteur. Les récepteurs 5-HT_{1B} sont localisés post-synaptiquement au niveau cérébral ainsi que sur les terminaisons nerveuses sympathiques périphériques, les vaisseaux sanguins cérébraux, les afférences primaires trigéminales (G.J. Molderings, *Naunyn-Schmiedeberg's Arch. Pharmacol*., **1990,** 342, 371 ; E. Hamel, *Mol*. *Pharmacol*., **1993,** 44, 242 ; A.T. Bruinvels, *Eur. J*. *Pharmacol.,* **1992,** 227, 357). Cette localisation suggère que par activation des populations de récepteurs 5-HT_{1B}, par un effet aussi bien vasculaire que neurogénique il est possible de soigner avec des agonistes les migraines et les céphalées. Avec des antagonistes, par action sur les récepteurs périphériques il sera possible de soigner des maladies du système cardiovasculaire telles que l'angor instable. D'autre part ces populations de récepteurs 5-HT_{1B} étant aussi présentes en fortes concentrations dans la come dorsale de la moelle épinière, le ganglion basal, l'hippocampe et les autres structures limbiques du cortex frontal (C. Del Arco, *Maunyn-Schmiedeberg's Arch. Pharmacol*., **1992**, 347, 248 ; S. Lowther, *Eur. J Pharmacol.,* **1992**, 222, 137 ; X Langlois, *J*. *Neurochem.,* **1995**, 65, 2671), peuvent être en partie responsables des troubles de l'humeur et du comportement et être impliquées dans les mécanismes de nociception. Du fait d'une double localisation, d'une part sur les neurones sérotoninergiques post-synaptiques, et d'autre part sur les corps cellulaires où ils jouent le rôle d'autorécepteur, on peut facilement conclure à leur implication dans la pathogénèse et par voie de conséquence en utilisant des ligands sélectifs de ces récepteurs, dans le traitement de la dépression, l'anxiété, les troubles impulsifs et les autres maladies psychiatriques liées à un dysfonctionnement de la transmission sérotoninergique (C. Waeber, *Neurochem. Res.,* **1990**, 15, 567; K. Herrick-Davis, *J*. *Neurochem*., **1988**, 51, 1906)

En ce qui concerne le récepteur 5-HT_{1B} (ex-5-HT_{1Dβ}) il est prédominant dans le système nerveux central de l'homme et du cobaye. De plus, seuls les récepteurs 5-HT_{1B} sont localisés comme autorécepteurs, ce qui n'est pas le cas des récepteurs 5-HT_{1D}(ex-5-HT_{1Dα}). Des ligands des récepteurs 5-HT_{1B}/5-HT_{1D} ont été décrits dans les demandes WO 96/00720 et WO 96/12713 : il s'agit de dérivés naphthylpipérazine. Des antagonistes des récepteurs 5-HT_{1B}/5-HT_{1D} de structure biphényliques ont aussi été décrits dans la demande WO 96/19477. Ces structures ne suggèrent en rien les composés de la présente invention.
Des dérivés de pipéridines et pipérazines substituées en position 4 par un hétérobicycle ont été décrits dans la demande EP 0 745 598 et le brevet US 5 464 834. Des dérivés de N-(1,4-benzodioxanyl)-pipérazines ont été décrits dans la demande EP 0 529 462.
La demande de brevet WO 95/07274 fait état de composés utilisés dans le traitement des maladies du système nerveux central et possédant une structure 4-amino pipéridine. Dans la formule générale l'azote extracyclique peut être relié, par l'intermédiaire d'une chaîne alkane, à des noyaux benzodioxane, tétrahydronaphtalène et chromane. Ces structures ne suggèrent nullement celles de la présente invention.
L'activité des produits de la présente invention a été démontrée au cours de nombreux tests biologiques et pharmacologiques.
La sélectivité pour les récepteurs 5-HT_{1B} a pu être évaluée in vitro au cours d'expériences de liaisons notamment vis à vis des récepteurs 5-HT_{1A}.
Le caractère agoniste ou antagoniste des produits de l'invention a pu être appréhendé grâce au test de l'hypothermie chez le cobaye (M. Stingle et al., *J*. *of Psychopharmacology*, **1994**, 8, 14).

Les expériences de microdialyse montrent l'intérêt des produits de la présente invention dans le traitement des différentes pathologies du système nerveux central. Réalisées dans le cortex frontal ces tests permettent d'envisager, dans le cas où les produits entraînent une augmentation de la libération de sérotonine, leur utilisation dans la dépression, les troubles impulsifs, l'obésité. S'ils produisent une diminution de la libération de sérotonine, ils seront utiles dans l'anxiété, les attaques de panique, les problèmes de sommeil, de cognition et l'abus de drogues. Enfin, s'ils produisent une augmentation de la dopamine et/ou noradrénaline, ils seront utiles dans la schizophrénie et comme ci-dessus dans la dépression et les problèmes cognitifs.

La présente invention a également pour objet les compositions pharmaceutiques contenant comme principe actif un composé de formule I ou un de ses sels physiologiquement tolérable mélangé ou associé à un ou plusieurs excipients pharmaceutiques appropriés.

Les compositions pharmaceutiques ainsi obtenues se présentent généralement sous forme dosée renfermant de 0,5 à 25 mg de principe actif. Elles peuvent, par exemple, revêtir la forme de comprimés, dragées, gélules, suppositoires, solutions injectables ou buvables et être administrées par la voie orale, rectale ou parentérale selon les formes utilisées.

La posologie varie selon l'âge et le poids du patient, la voie d'administration et les traitements associés et s'échelonne de 0,5 à 25 mg de principe actif, 1 à 3 fois par jour.

La présente invention a également pour objet le procédé de préparation des composés de formule I caractérisé en ce que :
- l'on condense un composé de formule II : 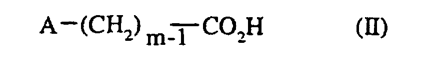
dans laquelle A et m ont les significations précédemment définies, avec une amine de formule III : 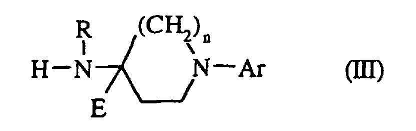 dans laquelle R, E, n et Ar ont les significations précédemment définies,
par l'intermédiaire du carbonyl diimidazole dans le dichlorométhane, pour obtenir un composé de formule IV : 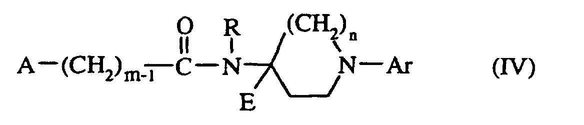 dans laquelle A. m, R, E, n et Ar ont les significations précédemment définies, que l'on réduit en composé de formule I, par l'intermédiaire du borane-diméthylsulfure dans le tétrahydrofurane.

Les composés de formule I dans laquelle R a la signification précédemment définie à l'exception de la valeur hydrogène ont également été préparés par transformation des composés de formule I dans laquelle R représente uniquement un atome d'hydrogène (c'est à dire par transformation des composés de formule I' : 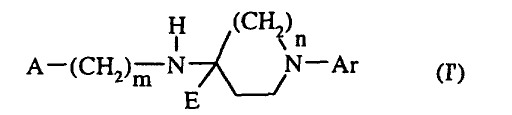 dans laquelle A, m, E, n et Ar ont les significations précédemment définies),
au moyen des méthodes classiques d'alkylation ou d'aralkylation en utilisant un halogénure un tosylate ou un mésylate adéquat de formule V :

RZ (V)

dans laquelle :
R a la signification précédemment définie et
Z représente un atome d'halogène,choisi parmi les atomes de chlore, brome et iode, un radical tosyloxy ou un radical mésyloxy.

Les composés de formule III sont préparés par action d'un ω,ω'-dihalogéno-3-amino alkane de formule VI 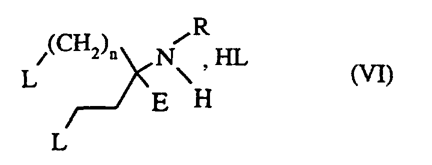 dans laquelle L est un atome de chlore, brome ou iode et R, E et n ont les significations précédemment définies sur une arylamine de formule VII :

ArNH₂ (VII)

dans laquelle Ar a la signification précédemment définie, dans un solvant tel que le chlorobenzène.

Si on le désire, on prépare les isomères optiques des composés de formule I renfermant un ou plusieurs carbones asymétriques, selon les méthodes classiques connues de la littérature.

Les sels des composés de formule I avec des acides pharmaceutiquement acceptables ont été obtenus selon des méthodes classiques comme indiqué dans les exemples ci-après.

Les matières premières sont soit des produits connus soit des produits obtenus à partir de substances connues, selon des procédés connus, comme décrit ci-après dans les préparations 1 à 6.

Les exemples suivants, donnés à titre non limitatif, illustrent la présente invention.

Les points de fusion ont été déterminés soit à la platine chauffante de Kofler (K), soit à la platine chauffante sous microscope (MK).

### Synthèse des matières premières

Les matières premières utilisées dans les exemples suivants ont été préparées comme suit :

### . Préparation 1 : 1-(2,3-dihydro[1,4]benzodioxin-5-yl)-4-amino pipéridine

### Stade 1 : 4-hydroxyimino tétrahydro-4H-pyrane

A température ambiante, on mélange 40,6g (0.406 mole) de tétrahydro-4H-pyran-4-one, 118.7g (1.71 mole) de chlorhydrate d'hydroxylamine et 118,1g (1.44 mole) d'acétate de sodium dans 810 ml d'éthanol. On porte à reflux pendant 20h, puis laisse refroidir. On filtre le solide, le rince à l'éthanol, puis concentre les filtrats. Le résidu est repris par 500 ml d'éther et agité énergiquement pendant 2h (produit insoluble blanchâtre très visqueux). On élimine l'insoluble par filtration et concentre le filtrat pour obtenir 49,5g du produit désiré (théorie : 46,7g) contenant 15% en poids d'acide acétique (Rendement corrigé : 90% environ), et qui est utilisé tel quel.

### Stade 2 : chlorhydrate du 4-amino tétrahydro-4H-pyrane

On mélange 49,3g (environ 0,405 mole) du composé obtenu au stade 1 et 15 ml de nickel de Raney dans 600 ml d'éthanol, puis on hydrogène le mélange à température ambiante sous 5.10⁵ Pa d'hydrogène pendant 4h. Après filtration du nickel de Raney, on ajoute 200 ml d'éther chlorhydrique-4,1N (environ 2 éq.), puis évapore les solvants pour recueillir 52,6g du produit désiré (théorie : 55,7g), qui est utilisé tel quel.

### Stade 3 : bromhydrate du 1,5-dibromo-3-amino pentane

A température ambiante, on dissout 52,3g (380 mmole) du composé obtenu au stade précédent dans 380 ml d'acide bromhydrique fumant (à 60%), puis on porte à reflux pendant 24h. On laisse refroidir, puis on ajoute 500 ml d'eau : un solide apparait après quelques minutes. On refroidit dans la glace, puis on filtre ce solide, le rince par très peu d'eau, puis le réempâte dans 200 ml d'éther, le filtre, le rince à l'éther et le sèche sous vide sur potasse. On obtient ainsi 69,5g du produit désiré (Rendement: 56 %) sous forme d'une poudre grise.

### Stade 4 : produit titre

A température ambiante, on mélange 20g (59,0 mmole) du composé obtenu au stade précédent et 8,9g (58,9 mmole) de 5-amino [1.4] benzodioxane dans 120 ml de chlorobenzène, puis on porte à reflux pendant la nuit. On laisse refroidir : le produit est déposé sur les parois du tricol. On décante la phase chlorobenzène, puis on reprend le résidu par 50 ml d'eau, puis 200ml d'acide chlorhydrique N. On lave à l'éther (émulsion importante), puis basifie à froid avec de la soude concentrée et extrait 3 fois par 200 ml d'acétate d'éthyle. Les phases organiques jointes sont séchées sur sulfate de magnésium. concentrées (15g), puis chromatographiées sur silice (éluant dichlorométhane/méthanol/ammoniaque : 95/5/0,5) pour donner 4,7g du produit désiré (théorie : 13,8g) sous forme d'une pâte.

### . Préparation 2 : acide 5,6-dihydro-8,9-diméthoxy pyrrolo [2,1-a] isoquinol-2-yl acétique

### Stade 1 : ester méthylique de l'acide 5,6-dihydro-8,9-diméthoxy pyrrolo [2,1-a] isoquinol-2-yl acétique

On porte à reflux, pendant 5h, 10,3g (50,0 mmole) de 1-méthyl-6,7-diméthoxy-3,4-dihydro isoquinoléine, 8,3g (55,0 mmole) de 4-chloro acétoacétate de méthyle et 12,6g (150 mmole) d'hydrogénocarbonate de sodium dans 100 ml d'éthanol. Après évaporation à sec, le résidu est repris par 250 ml de dichloroinéthane et lavé 2 fois par 100 ml d'eau. Après séchage de la phase organique sur sulfate de magnésium, puis concentration, le résidu (15,5g) est chromatographié sur 500g de silice (éluant : dichlorométhane) pour donner 8,5g du produit attendu (théorie : 15,1g).

### Stade 2 : produit titre

A température ambiante, sur 8,4g (27,9 mmole) du composé obtenu au stade précédent en suspension dans 55 ml de méthanol, on ajoute goutte à goutte 17 ml (34 mmole) de soude 2N et agite la nuit. Après évaporation à sec, le résidu est repris par 50 ml d'eau et lavé par 50 ml d'éther. La phase aqueuse est acidifiée par 50 ml d'acide chlorhydrique N et extraite 2 fois par 250 ml de dichlorométhane. Les phases organiques jointes sont séchées sur sulfate de magnésium, puis concentrées pour donner 7,5g du produit désiré (théorie : 8,0g).
P.F. (K) = 179°C

### . Préparation 3 : acide 5,6-dihydro pyrrolo[2,1-a] isoquinol-2-yl acétique

Obtenu comme à la préparation 2, mais en utilisant au stade 1 la 1-méthyl-3,4-dihydro isoquinoléine.
P.F. (K) = 75-85° C.

### . Préparation 4 : acide 8-chloro-5,6-dihydro-9-méthoxy pyrrolo[2,1-a] isoquinol-2-yl acétique

Obtenu comme à la préparation 2 mais en utilisant au stade 1 la 1-méthyl-6-chloro-7-méthoxy isoquinoléine.
P.F. (K) = 128° C.

### . Préparation 5 : acide 5,6-dihydro-8,9-diméthoxy pyrrolo [2,1-a] isoquinol-2-yl carboxylique

### Stade 1 : ester éthylique de l'acide 5,6-dihydro-8,9-diméthoxy pyrrolo [2,1-a] isoquinol-2-yl carboxylique

Obtenu de la même façon que le produit du stade 1 de la préparation 2 mais en utilisant le bromopyruvate d'éthyle à la place du 4-chloroacétoacétate de méthyle. Après purification du milieu réactionnel par chromatographie sur silice avec un éluant composé du mélange CH₂Cl₂/CH₃COOC₂H₅ 98/2, on obtient le produit attendu avec un rendement de 77 %.

### Stade 2 : Produit titre

Obtenu de la même façon que le produit du stade 2 de la préparation 2 mais en utilisant l'éthanol comme solvant à la place du méthanol. PF(MK) : 238-242 °C.

### . Préparation 6 : 1-(6-fluorochroman-8-yl)-4-amino pipéridine

### Stade 1 : 6-fluoro chromane 8-carboxaldéhyde

13,74 g (90,28 mmol) de 6-fluorochromane sont dissous dans 250 ml de chlorure de méthylène. On refroidit à 0 °C et coule goutte à goutte 20,15 ml (0,18 mol) de TiCl₄. La solution devient marron et on agite 10 minutes à température ambiante. On introduit alors 8.78 ml (99,3 mmol) d'α,α-dichlorométhyl éther. On agite la nuit à température ambiante. On verse sur de l'eau glacée et décante. La phase organique est séchée et évaporée pour conduire à 17,7 g d'un résidu qui est purifié par chromatographie sur silice (éluant CH₂Cl₂/cyclohexane 50/50). On obtient alors 6.3 g de produit attendu. Rendement : 38,7 %.

### Stade 2 : Acide 6-fluoro chromane 8-carboxylique

On dissout l'aldéhyde obtenue au stade précédent dans 52 ml d'acétone. On refroidit à 0 °C. On additionne lentement 17.43 ml de réactif de Jones tout en maintenant à une température inférieure à 10 °C. On laisse agiter 4 heures à température ambiante, évapore l'acétone, reprend avec 60 ml d'eau puis extrait à l'éther. Les phases éthérées sont extraites avec de la soude 1N. Les phases basiques sont acidifiées à l'acide chlorhydrique concentré et extraites à l'éther. On obtient 5,31 g de produit attendu. Rendement: 77,5 %.

### Stade 3 : N-(6-fluoro chroman-8-yl)carbamate de benzyle

On porte deux heures à 90 °C une solution composée de 137 ml de toluène, 5,3 g (27,07mmol) d'acide obtenu au stade précédent, 4,72 ml de triéthylamine et 7,29 ml (33,83 mmol) de diphényl phophorylazide. On ajoute ensuite, tout en maintenant à cette température, 3.52 ml d'alcool benzylique et laisse 20 heures à la même température. On lave à l'eau, à l'acide chlorhydrique 0,5 N, à nouveau à l'eau puis au bicarbonate de sodium et enfin à l'eau. On sèche et évapore pour obtenir 8,2 g du produit attendu.

### Stade 4 : 8-amino 6-fluoro chromane

8,1 g du composé obtenu au stade 3 sont dissous dans 80 ml d'éthanol. On hydrogène à pression ordinaire et température ambiante en présence de 0,39 g de paladium sur charbon. Après filtration et évaporation, on obtient 4,6 g d'un liquide qui correspond au produit attendu.

### Stade 5 : produit titre

2 g du composé obtenu au stade précédent sont traités comme décrit à préparation 1 stade 4. On obtient ainsi 1,1 g du produit désiré.

### EXEMPLE 1 : N-[1-(2,3-dihydro [1,4]benzodioxin-5-yl) pipérid-4-yl]-N-[2-(indan-1-yl) éthyl] amine et son hémifumarate

### Stade 1 : couplage acide/amine

A 0,75g (4,3 mmole) d'acide indan-1-yl acétique dans 15 ml de dichlorométhane, à température ambiante, on ajoute d'un trait 0,73g (4,5 mmole) de carbonyldiimidazole et agite pendant 4h à température ambiante. On ajoute alors rapidement 1,0g (4,3 mmole) de 1-(2,3-dihydro [1,4]benzodioxin-5-yl)-4-amino pipéridine (décrite à la préparation 1) en solution dans 5 ml de dichlorométhane et poursuit l'agitation à température ambiante pendant la nuit. On ajoute alors 100 ml de dichlorométhane et lave par 100 ml de soude N et 100 ml d'eau. Après séchage sur sulfate de magnésium et concentration, on obtient 1,55g du produit désiré (théorie : 1.7g).

### Stade 2 : réduction de l'amide

A température ambiante, sur 1,55g (3,9 mmole) du composé obtenu au stade précédent dans 15 ml de tétrahydrofurane, on ajoute goutte à goutte 1,12 ml (11,8 mmole) de borane-diméthyl sulfure dans 5 ml de tétrahydrofurane, puis porte à reflux la nuit. On laisse refroidir et, à température ambiante, on ajoute 7 ml de méthanol, puis porte de nouveau à reflux pendant 1h. On évapore à sec, reprend le résidu par de la soude N, puis extrait au dichlorométhane. Après séchage des phases organiques jointes sur sulfate de magnésium, puis concentration, le résidu (1,5g) est chromatographié sur 170g de silice (éluant : dichlorométhane/méthanol 98/2 à 90/10) pour donner 0,93g du produit attendu sous forme de base libre.
L'hémifumarate est obtenu dans l'éthanol, par addition d'un équivalent d'acide fumarique à 2% dans l'éthanol. Après filtration et séchage, puis recristallisation de l'éthanol, on obtient 0.41g du produit attendu. P.F. (K) : 189-192°C

### EXEMPLE 2 : N-[1-(2,3-dihydro [1.4] benzodioxin-5-yl) pipérid-4-yl]-N-(indan-1-yl méthyl) amine et son fumarate

Préparé comme le produit de l'exemple 1 mais en utilisant au stade 1 l'acide indan-1-yl carboxylique à la place de l'acide indan-1-yl acétique.
Le fumarate du produit titre est obtenu comme précédemment.
P.F. (M.K.) = 217-220° C

### EXEMPLE 3 : N-[1-(2,3-dihydro [1,4] benzodioxin-5-yl) pipérid-4-yl]-N-[3-(indan-1-yl) propyl] amine et son fumarate

Préparé comme le produit de l'exemple 1, mais en utilisant au stade 1 l'acide 3-(indan-1-yl) propionique à la place de l'acide indan-1-yl acétique.
Le fumarate du produit titre est obtenu comme à l'exemple 1, stade 2.
P.F. (M.K.) = 199-202° C

### EXEMPLE 4 : N-(benzocyclobuten-1-yl méthyl)-N-[1-(2,3-dihydro [1,4] benzodioxin-5-yl) pipérid-4-yl] amine et son fumarate

Préparé comme le produit de l'exemple 1 mais en utilisant au stade 1 l'acide benzocyclobuten-1-yl carboxylique à la place de l'acide indan-1-yl acétique.
Le fumarate est obtenu comme à l'exemple 1, stade 2.
P.F. (M.K.) = 224-226° C

### EXEMPLE 5 : N-[3-(benzocyclobuten-1-yl) propyl]-N-[1-(2,3-dihydro [1,4] benzodioxin-5-yl) pipérid-4-yl] amine et son fumarate

Préparé comme le produit de l'exemple 1 mais en utilisant au stade 1 l'acide 3-(benzocyclobuten-1-yl) propionique à la place de l'acide indan-1-yl acétique.

Le fumarate est obtenu comme à l'exemple 1, stade 2.
P.F. (M.K.) = 178-180° C

### EXEMPLE 6 : N-[2-(benzocyclobuten-1-yl)éthyl]-N-[1-(2,3-dihydro [1,4] benzodioxin-5-yl) pipérid-4-yl] amine et son fumarate

Préparé comme le produit de l'exemple 1 mais en utilisant au stade 1 l'acide benzocyclobuten-1-yl acétique à la place de l'acide indan-1-yl acétique.
Le fumarate est préparé comme à l'exemple 1, stade 2.
P.F. (M.K.) = 186-188° C

### EXEMPLE 7 : N-[4-(benzocyclobuten-1-yl) butyl]-N-[1-(2,3-dihydro [1,4] benzodioxin-5-yl) pipérid-4-yl] amine et son fumarate

Préparé comme le produit de l'exemple 1 mais en utilisant au stade 1 l'acide 4-(benzocyclobuten-1-yl) butyrique à la place de l'acide indan-1-yl acétique.
Le fumarate est obtenu comme à l'exemple 1, stade 2.
P.F. (M.K.) = 201-205° C

### EXEMPLE 8 : N-[1-(2,3-dihydro [1,4] benzodioxin-5-yl) pipérid-4-yl]-N-(4,5-diméthoxy benzocyclobuten-1-yl méthyl) amine et son fumarate

Préparé comme le composé de l'exemple 1 mais en utilisant au stade 1 l'acide 4,5-diméthoxy benzocyclobuten-1-yl carboxylique à la place de l'acide indan-1-yl acétique.
Le fumarate est obtenu comme à l'exemple 1, stade 2.
P.F. (M.K.) = 218-222° C

### EXEMPLE 9 : N-[2-(adamant-1-yl) éthyl]-N-[1-(2,3-dihydro [1,4] benzodioxin-5-yl) pipérid-4-yl] amine et son fumarate

Préparé comme décrit dans l'exemple 1 mais en utilisant au stade 1 l'acide adamant-1-yl acétique à la place de l'acide indan-1-yl acétique.
Le fumarate est obtenu comme à l'exemple 1, stade 2.
P.F. (M.K.) = 251-254° C

### EXEMPLE 10 : N-[adamant-1-yl méthyl)-N-[1-(2,3-dihydro [1,4] benzodioxin-5-yl) pipérid-4-yl] amine et son fumarate

Préparé comme décrit dans l'exemple 1 mais en utilisant au stade 1 l'acide adamant-1-yl carboxylique à la place de l'acide indan-1-yl acétique.
Le fumarate est obtenu comme à l'exemple 1, stade 2.
P.F. (M.K.) = 239-243° C

### EXEMPLE 11 : 5,6-dihydro-8,9-diméthoxy-2-{2-{[1-(2,3-dihydro [1,4] benzodioxin-5-yl) pipérid-4-yl amino]éthyl}}pyrrolo[2,1-a] isoquinoléine et son hémifumarate

Préparé comme décrit dans l'exemple 1 mais en utilisant au stade 1 l'acide 5,6-dihydro 8.9-diméthoxy pyrrolo [2,1-a] isoquinol-2-yl acétique (préparation 4) à la place de l'acide indan-1-yl acétique.
L'hémifumarate est obtenu comme à l'exemple 1, stade 2.
P.F. (M.K.) = 208-213° C

### EXEMPLE 12 : 5,6-dihydro-2-{2-{[1-(2,3-dihydro [1,4] benzodioxin-5-yl) pipérid-4-yl amino]éthyl}} pyrrolo[2,1-a] isoquinoléine et son hémifumarate

Préparé comme décrit dans l'exemple 1 mais en utilisant au stade 1 l'acide 5,6-dihydro pyrrolo [2,1-a] isoquinol-2-yl acétique (préparation 3) à la place de l'acide indan-1-yl acétique.
L'hémifumarate est obtenu selon la méthode de l'exemple 1, stade 2.
P.F. (M.K.) = 221-225° C

### EXEMPLE 13 : 5,6-dihydro-9-méthoxy-2-{2-{[1-2,3-dihydro [1.4] benzodioxin-5-yl) pipérid-4-yl amino] éthyl}} pyrrolo[2,1-a] isoquinoléine et son hémifumarate

Préparé comme décrit dans l'exemple 1 mais en utilisant au stade 1 l'acide 8-chloro-5,6-dihydro-9-méthoxy pyrrolo [2,1-a] isoquinol-2-yl acétique (préparation 4) à la place de l'acide indan-1-yl acétique.
L'hémifumarate est obtenu selon la méthode de l'exemple 1, stade 2.
P.F. (M.K.) = 252-255° C

### EXEMPLE 14 : 1-(2,3-dihydro [1,4] benzodioxin-5-yl)-4-{N-benzyl-2-(indan-1-yl) éthylamino} pipéridine et son fumarate

A température ambiante, on mélange 1,0g (2,6 mmole) de la base libre du produit obtenu à l'exemple 1 stade 2, 0,31 ml (2,6 mmole)de bromure de benzyle, 1,46g (10,6 mmole) de carbonate de potassium et 30 ml d'acétone. On porte à reflux pendant 24h, puis évapore à sec, reprend le résidu par 100 ml d'acétate d'éthyle et lave 2 fois par 50 ml d'eau. Après séchage sur sulfate de magnésium et concentration, on obtient 1,23g du produit attendu sous forme de base libre.
Le fumarate correspondant est obtenu dans l'éthanol, par addition d'un équivalent d'une solution d'acide fumarique à 2% dans l'éthanol. Après filtration et séchage, on obtient 0,99g du produit attendu.
P.F. (K.) : 125-128°C

### EXEMPLE 15 : 5,6-dihydro-8,9-diméthoxy-2-{[1-(2,3-dihydro [1,4] benzodioxin-5-yl)pipérid-4-yl amino]méthyl}pyrrolo [2,1-a] isoquinoléine et son hémifumarate

Le produit titre sous forme de base a été prepare comme décrit dans l'exemple 1 mais en utilisant a stade 1 l'acide 5,6-dihydro-8,9-diméthoxy pyrrolo [2,1-a] isoquinol-2-yl carboxylique (décrit à la préparation 5) à la place de l'acide indan 1-yl acétique.
L'hémifumarate correspondant a été obtenu comme à l'exemple 1, stade 2.
PF(MK) : 244-246 °C.

### EXEMPLE 16 : N-[2-(adamant-2-yl)éthyl]-N-[1-(2,3-dihydro [1,4] benzodioxin-5-yl) pipérid-4-yl]amine et son fumarate

Le produit titre sous forme de base a été préparé comme décrit dans l'exemple 1. mais en utilisant au stade 1 l'acide adamant-2-yl acétique à la place de l'acide indan-1-yl acétique.
Le fumarate correspondant a été obtenu comme à l'exemple 1, stade 2. PF(MK) : 226-230 °C.

### EXEMPLE 17 : N-[2-(adamant-1-yl)éthyl]-N-[1-(chroman-8-yl) pipérid-4-yl] amine et son fumarate

Le produit titre sous forme de base a été préparé comme décrit dans l'exemple 1, mais en utilisant au stade 1 la 1-(chroman-8-yl) 4-amino pipéridine à la place de la 1-(2,3-dihydro [1.4]-benzodioxin-5-yl)-4-amino pipéridine et l'acide adamant-1-yl acétique à la place de l'acide indan-1-yl acétique.
Le fumarate correspondant a été obtenu comme à l'exemple 1 stade 2. PF (MK) : 252-256 °C.

### EXEMPLE 18 : 5,6-dihydro-8,9-diméthoxy-2-{2-[1-(chroman-8-yl)pipérid-4-yl amino ]éthyl} pyrrolo [2,1-a] isoquinoléine et son hémifumarate

Le produit titre sous forme de base a été préparé comme décrit dans l'exemple 1 mais en utilisant au stade 1 la 1-(chroman-8-yl)-4-amino pipéridine à la place du composé de la préparation 1 et l'acide 5,6-dihydro-8,9-diméthoxy pyrrolo [2,1-a] isoquinol-2-yl acétique à la place de l'acide indan-1-yl acétique.
L'hémifumarate correspondant a été obtenu comme à l'exemple 1, stade 2.
PF(MK) : 231-235 °C.

### EXEMPLE 19 : 5,6-dihydro-8,9-diméthoxy-2-{2-[1-(6-fluorochroman-8-yl) pipérid-4-yl amino]éthyl} pyrrolo [2,1-a] isoquinoléine et son hémifumarate.

Le produit titre sous forme de base a été préparé comme décrit dans l'exemple 1. mais en utilisant au stade 1 la 1-(6-fluorochroman-8-yl)-4-amino pipéridine (décrite à la préparation 6) à la place du composé de la préparation 1 et l'acide 5,6-dihydro-8,9-diméthoxy pyrrolo [2,1-a] isoquinol-2-yl acétique à la place de l'acide indan-2-yl acétique.
L'hémifumarate correspondant à été obtenu comme à l'exemple 1 stade 2.
PF (MK) : 215-218 °C.

### EXEMPLE 20 : ETUDE PHARMACOLOGIQUE

### ◆ Etudes de liaison

### . Liaison 5-HT_{1B}

### a) Préparation des membranes

Après dissection des cerveaux de cobaye, les striatums extraits sont congelés puis homogénéisés dans 20 volumes (poids/volume) de Tris-HCl 50mM (pH 7.7 à température ambiante) contenant 4mM de CaCl₂, 0,1% d'acide ascorbique et centrifugées à 48.000g pendant 25 minutes à 4° C. Le surnageant est séparé et le précipité est remis en suspension dans le même volume de tampon avant d'être incubé à 37° C pendant 15 minutes pour retirer la sérotonine endogène. Finalement la suspension est centrifugée à 48.000g pendant 25 minutes à 4° C et le précipité remis en suspension dans 80 volumes de tampon qui contient 10 µM de pargylline.

### b) Etude de liaison

Les études de liaison ([³H]-GR 125743) sont effectuées en triple dans le tampon suivant : 50 mM Tris-HCl (pH 7,7 à température ambiante) contenant 4mM de CaCl₂, 0,1 % d'acide ascorbique et 10 µM de pargylline. Le volume final de 500 µl est constitué de 100 µl de radioligand, 100 µl de tampon ou de composé à tester et 300 µl de membranes. La sérotonine (10 µM) est utilisée pour définir la liaison non spécifique. Dans les expériences de compétition, la concentration de [³H]-GR 125743 est de 1 nM. Les incubations sont initiées par l'addition de la préparation membranaire et durent 60 minutes à température ambiante. La réaction est stoppée par filtration rapide au travers de filtres Whatman GF/B pré-traités avec 0.1% de polyéthylènimine, suivi par trois rinçages avec du tampon froid. La liaison spécifique représente environ 90% de la liaison totale aux concentrations de radioligand proche de la valeur du Kd.

### . Analyses de données

Les données sont analysées par régression non linéaire en utilisant le programme PRISM (Graphpad Software Inc., San Diego, CA) pour déterminer les valeurs du Kd (constante de dissociation du radioligand) et du Bmax (nombre de sites maximum) pour les expériences de saturation et celles de l'IC₅₀ (concentration inhibitrice 50) et du nombre de Hill pour les expériences de compétition. La constante d'inhibition (Kᵢ) est calculée selon l'équation de Cheng-Prussof : Kᵢ = IC₅₀/1+L_{/Kd} dans laquelle L représente la concentration du radioligand.
Les résultats sont exprimés en pKᵢ = - log Kᵢ.
Les composés de la présente invention montrent une très bonne affinité pour le récepteur 5-HT_{1B}. A titre d'exemple le pKᵢ du composé de l'exemple 9 est de 8,0.

### . Liaison 5-HT_{1A}

Les études de liaison sur le récepteur 5-HT_{1A} ont été effectuées selon les méthodes connues et décrites dans la littérature (cf. S.J. Peroutka, *J*. *Neurochem*., **1986**, 47, 529-40, M.J. Millan. *J*. *Pharmacol*. *Exp*. *Ther*., **1994**, 268, 337-52). Les résultats sont aussi exprimés en pKᵢ.

Les composés de la présente invention sont très peu affins pour le récepteur 5HT_{1A}. A titre d'exemple le pKᵢ du composé de l'exemple 4 est de 5,5.

Ceci démontre l'excellente sélectivité des produits de l'invention.

### ◆ Test de l'hypothermie chez le cobaye

- Les-cobayes sont stockés en batterie par trois, avec libre accès à la nourriture et à l'eau. pendant une semaine avant d'entrer dans l'étude. Une heure avant chaque expérience, les cobayes sont placés dans des cages individuelles avec libre accès à l'eau. Ils sont remis dans leur batterie respective à la fin de chaque expérience. Les mesures de température sont effectuées à l'aide d'un thermomètre digital et d'une sonde rectale. Chaque cobaye est pesé, on prend sa température corporelle basale puis on injecte i.p. ou p.o. le composé de la présente invention à évaluer.

Dans le cas d'un agoniste, on prend la température corporelle de chaque cobaye toutes les 30 minutes pendant 2 heures.

Dans le cas d'un antagoniste, 15 minutes après son injection, on injecte à nouveau chaque cobaye i.p. avec l'agoniste prototypique 5-HT_{1B} : GR46611 (5mg/kg/ml). On prend alors la température pendant 2 heures toutes les 30 minutes.

Le critère de jugement utilisé est la différence de température à un temps donné par rapport à la température basale. Pour chaque dose de produit et pour chaque temps (t₃₀, t₆₀, t₉₀, t₁₂₀) on calcule la moyenne et l'erreur standard à la moyenne.

A titre d'exemple et pour illustrer les effets des produits de l'invention à t₉₀ et t₁₂₀ et par voie i.p.. on rapporte dans le tableau suivant les résultats du composé de l'exemple 11 qui se comporte comme un antagoniste.

| INJECTION 1 (a) | INJECTION 2 (b) | ΔT°C (90 min)(b) | ΔT°C (120 min)(b) |
|---|---|---|---|
| Véhicule | Véhicule | 0±0,1 | 0±0,1 |
| Véhicule | GR46611 (5 mg/kg) | -1,05±0,13 | -1,40±0,36 |
| **Produit de l'exemple 11** 0.04 mg/kg | GR46611 (5 mg/kg) | -1,10±0.36 | -1,43±0,24 |
| **Produit de l'exemple 11** 0,16 mk/kg | GR46611 (5 mg/kg) | -0,57±0,17 | -0,67±0,28 |
| **Produit de l'exemple 11** 0,63 mg/kg | GR46611 (5 mg/kg) | -0,37*±0,14 | 0,50*±0,18 |

| | | | |
|---|---|---|---|
| (a) : voie i.p. | | | |
| (b) les valeurs sont les moyennes ± sem N≥6 par valeur | | | |
| *p < 0,05 versus véhicule/GR46611 selon le test de Dunett | | | |

### ◆ Expérience de microdialyse chez le rat

Les rats sont anesthésiés au pentobarbital (60mg/kg i.p.). Ils sont placés dans un appareil stéréotaxique de Kopf et le guide de la canule est implanté dans le cortex frontal cingulé suivant les coordonnées décrites comme suit dans l'atlas de Paxinos et Watson (1982) : AP : + 2.2. L : ± 0,6, DV : - 0,2. Les rats sont mis en cage séparément et ne sont utilisés en dialyse que 5 jours plus tard. Le jour de la dialyse la sonde est descendue lentement et maintenue dans sa position. La sonde est perfusée à un débit de 1 ml/min avec une solution de 147,2 mM de NaCl, 4 mM de KCl et 2,3 mM de CaCl₂ amené à pH 7,3 avec un tampon phosphate (0.1 M). Deux heures après l'implantation, les échantillons sont collectés toutes les 20 minutes pendant 4 heures. Trois échantillons de base sont collectés avant l'administration des produits à tester. Les rats sont laissés dans leur cage individuelle pendant toute l'expérience. A la fin de l'expérience, les rats sont décapités et le cerveau prélevé est congelé dans l'isopentane. Des sections d'une épaisseur de 100 µm sont coupées et colorées avec du cresyl violet, ce qui permet la vérification de l'emplacement des sondes.

La quantification simultanée de dopamine, norépinéphrine et sérotonine est effectuée de la façon suivante : 20 µl d'échantillons de dialyse sont dilués avec 20 µl de phase mobile (NaH₂PO₄ : 75 mM, EDTA : 20 µM, sodium dodecanesulphonate : 1 mM, méthanol : 17,5%, triethylamine : 0.01%, pH : 5,70) et 33 µl sont analysés par HPLC avec une colonne en phase inverse (hypersil ODS 5 µm, C18, 150 x 4,6 mm, Thermo Separation Products, Les Ulis, France) thermostatée à 45° C et quantifiés par l'intermédiaire d'un détecteur coulométrique. Le potentiel de la première électrode du détecteur est fixée à - 90 mV (réduction) et la seconde à + 280 mV (oxydation). La phase mobile est injectée avec une pompe Beckman 116 à un débit de 2ml/min. Les limites de sensibilité pour la dopamine, la norépinéphrine et la sérotonine sont de 0.55 fmole par échantillon. Tous les produits de l'invention sont injectés par voie sous cutanée dans un volume de 1,0 ml/kg. Les produits sont dissous dans de l'eau distillée additionnée de quelques gouttes d'acide lactique si nécessaire. Les quantités de neurotransmetteurs sont exprimées comme une fonction de la moyenne des trois valeurs de base. Une analyse de variance, avec le facteur temps comme mesure répétée, suivi d'un test de Newman-Keuls (P < 0,05) est utilisée pour l'évaluation statistique des effets des produits.

Dans le cas des agonistes on a observé une diminution de la concentration extracellulaire de sérotonine.

Dans le cas des antagonistes on a observé la réversion de la diminution de la concentration extracellulaire de sérotonine produite par l'agoniste GR46611 injecté 20 minutes après les composés de l'invention à tester.

## Revendications

1. Les composés de N-aryl pipéridine de formule I : 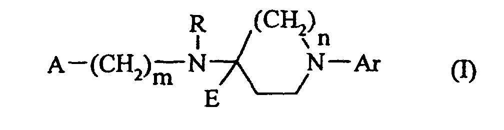 dans laquelle :
. m représente un nombre entier de 1 à 5 inclus ;
. n représente 2 ;
. R représente un atome d'hydrogène ou un radical aralkyle dans lequel la partie alkyle renferme de 1 à 5 atomes de carbone en chaine droite ou ramifiée ;
. E représente un atome d'hydrogène;
. Ar représente : 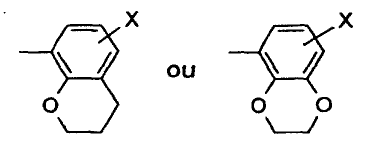 X étant un atome d'hydrogène
ou d'halogène et
. A représente : 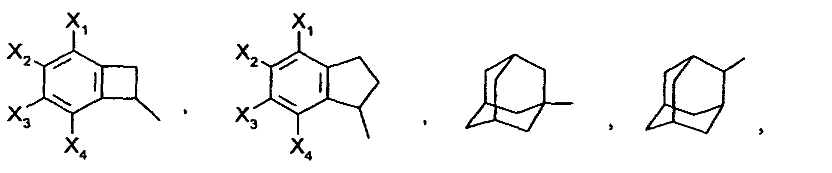 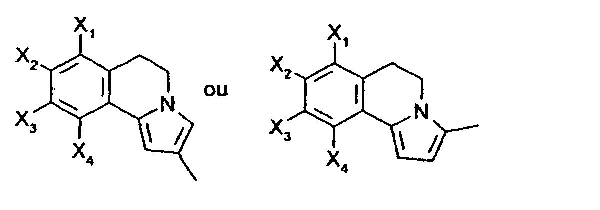
dans lesquels :
. X₁, X₂, X₃ et X₄, identiques ou différents représentent
. chacun un atome d'hydrogène ou d'halogène ou un radical (C₁-C₅)alkoxy en chaîne droite ou ramifiée,
sous forme de mélange racémique et d'isomères optiques quand ils existent,
et leurs sels d'addition acides physiologiquement tolérables.

2. Un composé de la revendication 1 choisi parmi le groupe constitué par les
N-[1-(2,3-dihydro [1,4]benzodioxin-5-yl) pipérid-4-yl]-N-[2-(indan-1-yl) éthyl] amine et son hémifumarate,
N-[1-(2,3-dihydro [1,4] benzodioxin-5-yl) pipérid-4-yl]-N-(indan-1-yl méthyl) amine et son fumarate,
N-[1-(2,3-dihydro [1,4] benzodioxin-5-yl) pipérid-4-yl]-N-[3-(indan-1-yl) propyl] amine et son fumarate,
N-(benzocyclobuten-1-yl méthyl)-N-[1-(2,3-dihydro [1,4] benzodioxin-5-yl) pipérid-4-yl] amine et son fumarate,
N-[3-(benzocyclobuten-1-yl) propyl]-N-[1-(2,3-dihydro [1,4] benzodioxin-5-yl) pipérid-4-yl] amine et son fumarate,
N-[2-(benzocyclobuten-1-yl) éthyl]-N-[1-(2,3-dihydro [1,4] benzodioxin-5-yl) pipérid-4-yl] amine et son fumarate,
N-[4-(benzocyclobuten-1-yl) butyl]-N-[1-(2,3-dihydro [1,4] benzodioxin-5-yl) pipérid-4-yl] amine et son fumarate,
N-[1-(2,3-dihydro [1,4] benzodioxin-5-yl) pipérid-4-yl]-N-(4.5-diméthoxy benzocyclobuten-1 - yl méthyl) amine et son fumarate,
N-[2-(adamant-1-yl) éthyl]-N-[1-(2,3-dihydro [1,4] benzodioxin-5-yl) pipérid-4-yl] amine et son fumarate,
N-[adamant-1-yl méthyl)-N-[1-(2,3-dihydro [1,4] benzodioxin-5-yl) pipérid-4-yl] amine et son fumarate,
5,6-dihydro-8,9-diméthoxy-2-{2-{[1-(2,3-dihydro [1,4] benzodioxin-5-yl) pipérid-4-yl amino] éthyl}} pyrrolo[2,1-a] isoquinoléine et son hémifumarate,
5.6-dihydro-2-{2-{[1-(2,3-dihydro [1,4] benzodioxin-5-yl) pipérid-4-yl amino] éthyl}} pyrrolo[2,1-a] isoquinoléine et son hémifumarate,
5,6-dihydro-9-méthoxy-2-{2-{[1-(2,3-dihydro [1,4] benzodioxin-5-yl) pipérid-4-yl amino] éthyl}} pyrrolo[2,1-a] isoquinoléine et son hémifumarate,
1-(2,3-dihydro [1,4] benzodioxin-5-yl)-4-{N-benzyl-2-(indan-1-yl) éthylamino} pipéridine et son fumarate,
5,6-dihydro-8,9-diméthoxy-2-{[1-(2,3-dihydro [1,4] benzodioxin-5-yl)pipérid-4-yl amino] méthyl}pyrrolo [2,1-a] isoquinoléine et son hémifumarate,
N-[2-(adamant-2-yl)éthyl]-N-[1-(2,3-dihydro [1,4] benzodioxin-5-yl) pipérid-4-yl]amine et son fumarate,
N-[2-(adamant-1-yl)éthyl]-N-[1-(chroman-8-yl) pipérid-4-yl] amine et son fumarate,
5,6-dihydro-8,9-diméthoxy-2-{2-[1-(chroman-8-yl)pipérid-4-yl amino]éthyl) pyrrolo [2,1-a] isoquinoléine et son hémifumarate, et
5,6-dihydro-8,9-diméthoxy-2-{2-[1-(6-fluorochroman-8-yl) pipérid-4-yl amino]éthyl} pyrrolo [2,1-a] isoquinoléine et son hémifumarate.

3. Un composé selon les revendications 1 et 2 qui est la N-(benzocyclobuten-1-yl méthyl)-N-[1-(2,3-dihydro [1,4] benzodioxin-5-yl) pipérid-4-yl] amine et son fumarate.

4. Un composé selon les revendications 1 et 2 qui est la N-[2-(adamant-1-yl) éthyl]-N-[1-(2,3-dihydro [1,4] benzodioxin-5-yl) pipérid-4-yl] amine et son fumarate.

5. Un composé selon les revendications 1 et 2 qui est la 5,6-dihydro-8,9-diméthoxy-2-{2-{[1-(2,3-dihydro [1,4] benzodioxin-5-yl) pipérid-4-yl amino] éthyl}} pyrrolo[2,1-a] isoquinoléine et son hémifumarate.

6. Le procédé de préparation des composés de la revendication 1 **caractérisé en ce que** :
- l'on condense un composé de formule II :
A-(CH₂)ₘ₋₁-CO₂H (II)
dans laquelle A et m ont les significations définies dans la revendication 1, avec une amine de formule III : 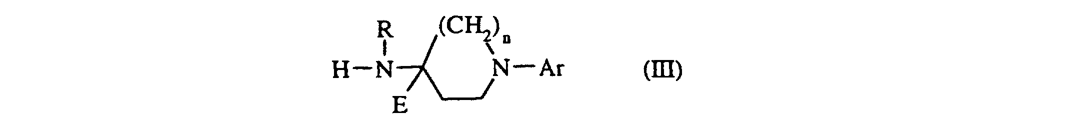 dans laquelle R, E, n et Ar ont les significations définies dans la revendication 1,
par l'intermédiaire du carbonyl diimidazole dans le dichlorométhane, pour obtenir un composé de formule IV : 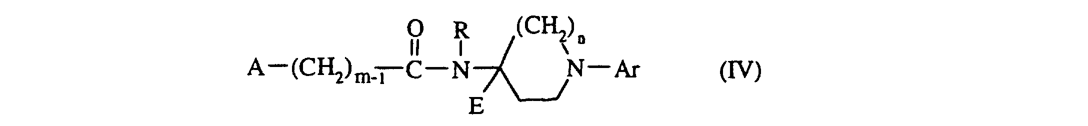 dans laquelle A, m, R, E, n et Ar ont les significations précédemment définies, que l'on réduit en composé de formule I, par l'intermédiaire du borane-diméthylsulfure dans le tétrahydrofurane,
si on le désire, on prépare les isomères des composés de formule I renfermant un ou plusieurs carbones asymétriques, selon les méthodes classiques connues de la littérature, et le cas échéant, les composés I ainsi obtenus sont traités avec des acides pharmaceutiquement acceptables pour obtenir les sels d'addition acides correspondants.

7. Les compositions pharmaceutiques, utilisables dans le traitement des maladies psychiatriques, des maladies dégénératives, de la douleur, de la migraine, des céphalées, des accidents vasculaires cérébraux, de la boulimie, de l'anorexie, de l'abus de drogue et des maladies cardiovasculaires, contenant un composé selon une des revendications 1 à 5, avec un ou plusieurs excipients pharmaceutiques appropriés.

## Patentansprüche

1. N-Aryl-piperidin-Verbindungen der Formel (I): 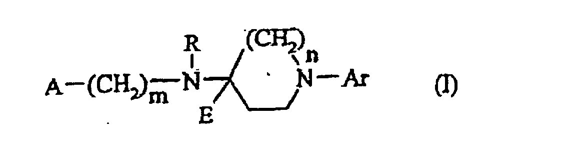 in der:
. m eine ganze Zahl mit einem Wert von 1 bis 5 einschließlich bedeutet;
. n 2 bedeutet;
. R ein Wasserstoffatom oder eine Aralkylgruppe, in der der Alkylteil 1 bis 5 Kohlenstoffatome in gerader oder verzweigter Kette umfaßt, bedeutet;
. E ein Wasserstoffatom darstellt;
. Ar: 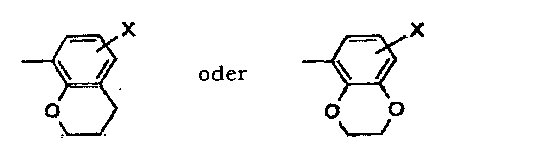 darstellt,
worin X ein Wasserstoffatom oder ein Halogenatom bedeutet und
. A: 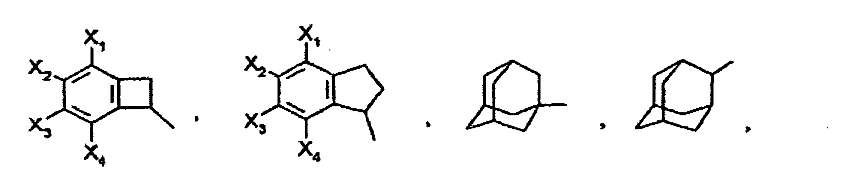 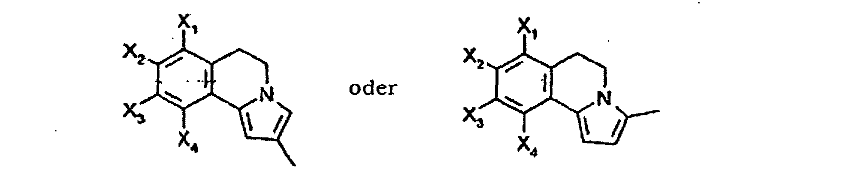 bedeutet, in denen:
. X₁, X₂, X₃ und X₄, die gleichartig oder verschieden sind,
. jeweils ein Wasserstoffatom oder ein Halogenatom oder eine (C₁-C₅)-Alkoxygruppe mit gerader oder verzweigter Kette bedeuten,
in Form der racemischen Mischung und von optischen Isomeren, falls diese existieren,
und deren Additionssalze mit physiologisch verträglichen Säuren.

2. Verbindung nach Anspruch 1, ausgewählt aus der Gruppe, die die folgenden Verbindungen umfaßt:
N-[1-(2,3-Dihydro[1,4]benzodioxin-5-yl)-piperid-4-yl]-N-[2-(indan-1-yl)-ethyl]-amin und dessen Hemifumarat,
N-[1-(2,3-Dihydro[1,4]benzodioxin-5-yl)-piperid-4-yl]-N-(indan-1-yl-methyl)-amin und dessen Fumarat,
N-[1-(2,3-Dihydro[1,4]benzodioxin-5-yl)-piperid-4-yl]-N-[3-(indan-1-yl)-propyl]-amin und dessen Fumarat,
N-(Benzocyclobuten-1-yl-methyl)-N-[1-(2,3-dihydro[1,4]benzodioxin-5-yl)-piperid-4-yl]-amin und dessen Fumarat,
N-[3-(Benzocyclobuten-1-yl)-propyl]-N-[1-(2,3-dihydro[1,4]benzodioxin-5-yl)-piperid-4-yl]-amin und dessen Fumarat,
N-[2-(Benzocyclobuten-1-yl)-ethyl]-N-[1-(2,3-dihydro[1,4]benzodioxin-5-yl)-piperid-4-yl]-amin und dessen Fumarat,
N-[4-(Benzocyclobuten-1-yl)-butyl]-N-[1-(2,3-dihydro[1,4]benzodioxin-5-yl)-piperid-4-yl]-amin und dessen Fumarat,
N-[1-(2,3-Dihydro[1,4]benzodioxin-5-yl)-piperid-4-yl]-N-(4,5-dimethoxybenzocyclobuten-1-yl-methyl)-amin und dessen Fumarat,
N-[2-(Adamant-1-yl)-ethyl]-N-[1-(2,3-dihydro[1,4]benzodioxin-5-yl)-piperid-4-yl]-amin und dessen Fumarat,
N-[Adamant-1-yl-methyl)-N-[1-(2,3-dihydro[1,4]benzodioxin-5-yl)-piperid-4-yl]-amin und dessen Fumarat,
5,6-Dihydro-8,9-dimethoxy-2-{2-{[1-(2,3-dihydro[1,4]benzodioxin-5-yl)-piperid-4-yl-amino]-ethyl}}-pyrrolo[2,1-a]isochinolein und dessen Hemifumarat,
5,6-Dihydro-2-{2-{[1-(2,3-dihydro[1,4]benzodioxin-5-yl)-piperid-4-yl-amino]-ethyl}}-pyrrolo[2,1-a]-isochinolein und dessen Hemifumarat,
5,6-Dihydro-9-methoxy-2-{2-{[1-(2,3-dihydro[1,4]benzodioxin-5-yl)-piperid-4-ylamino]-ethyl}}-pyrrolo[2,1-a]isochinolein und dessen Hemifumarat.
1-(2,3-Dihydro[1,4]benzodioxin-5-yl)-4-{N-benzyl-2-(indan-1-yl)-ethylamino)-piperidin und dessen Fumarat,
5,6-Dihydro-8,9-dimethoxy-2-{[1-(2,3-dihydro[1,4]benzodioxin-5-yl)-piperid-4-yl-amino]-methyl}-pyrrolo[2,1-a]isochinolein und dessen Hemifumarat,
N-[2-(Adamant-2-yl)-ethyl]-N-[1-(2,3-dihydro[1,4]benzodioxin-5-yl)-piperid-4-yl]-amin und dessen Fumarat,
N-[2-(Adamant-1-yl)-ethyl]-N-[1-(chroman-8-yl)-piperid-4-yl]-amin und dessen Fumarat,
5,6-Dihydro-8,9-dimethoxy-2-{2-[1-(chroman-8-yl)-piperid-4-yl-amino]-ethyl}-pyrrolo[2,1-a]isochinolein und dessen Hemifumarat, und
5,6-Dihydro-8,9-dimethoxy-2-{2-[1-(6-fluorchroman-8-yl)-piperid-4-yl-amino]-ethyl}-pyrrolo[2,1-a]isochinolein und dessen Hemifumarat.

3. Verbindung nach den Ansprüchen 1 und 2, nämlich N-(Benzocyclobuten-1-yl-methyl)-N-[1-(2,3-dihydro[1,4]benzodioxin-5-yl)-piperid-4-yl]-amin und dessen Fumarat.

4. Verbindung nach den Ansprüchen 1 und 2, nämlich N-[2-(Adamant-1-yl)-ethyl]-N-[1-(2,3-dihydro[1,4]benzodioxin-5-yl)-piperid-4-yl]-amin und dessen Fumarat.

5. Verbindung nach den Ansprüchen 1 und 2, nämlich 5,6-Dihydro-8,9-dimethoxy-2-{2-{[1-(2,3-dihydro[1,4]benzodioxin-5-yl)-piperid-4-yl-amino]-ethyl}}-pyrrolo[2,1-a]isochinolein und dessen Hemifumarat.

6. Verfahren zur Herstellung der Verbindungen nach Anspruch 1, **dadurch gekennzeichnet, daß** man:
- eine Verbindung der Formel II:
A-(CH₂)ₘ₋₁-CO₂H (II)
in der A und m die in Anspruch 1 angegebenen Bedeutungen besitzen, mit einem Amin der Formel III: 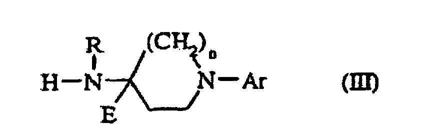 in der R, E, n und Ar die in Anspruch 1 angegebenen Bedeutungen besitzen,
mit Hilfe von Carbonyldiimidazol in Dichlormethan kondensiert zur Bildung einer Verbindung der Formel IV: 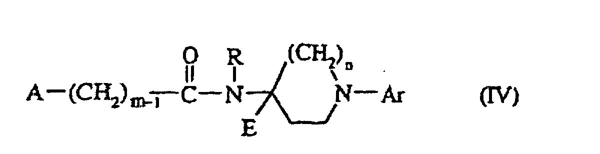 in der A, m, R, E, n und Ar die oben angegebenen Bedeutungen besitzen, welche man mit Hilfe von Boran-Dimethylsulfid in Tetrahydrofuran zu der Verbindung der Formel I reduziert, man gewünschtenfalls die Isomeren der Verbindungen der Formel I, die ein oder mehrere asymmetrische Kohlenstoffatome enthalten, mit Hilfe von aus der Literatur bekannten klassischen Methoden herstellt und gewünschtenfalls die in dieser Weise erhaltenen Verbindungen I mit pharmazeutisch annehmbaren Säuren behandelt zur Bildung der entsprechenden Säureadditionssalze.

7. Pharmazeutische Zubereitung für die Behandlung von psychiatrischen Krankheiten, degenerativen Erkrankungen, Schmerzen, Migräne, Kopfschmerzen, Zerebralgefäßvorfällen, Schlaganfällen, der Bulimie, der Anorexie, des Drogenmißbrauchs und von kardiovaskulären Erkrankungen, enthaltend eine Verbindung nach einem der Ansprüche 1 bis 5 zusammen mit einem oder mehreren pharmazeutisch geeigneten Trägermaterialien.

## Claims

1. N-arylpiperidine compounds of formula I : 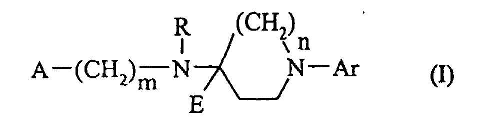 wherein :
. m represents an integer from 1 to 5 inclusive ;
. n represents 2 ;
. R represents a hydrogen atom or an aralkyl radical in which the alkyl moiety contains from 1 to 5 carbon atoms in straight or branched chain;
. E represents a hydrogen atom ;
. Ar represents : 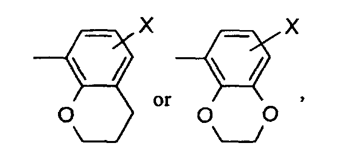 X being a hydrogen or
halogen atom
. and A represents : 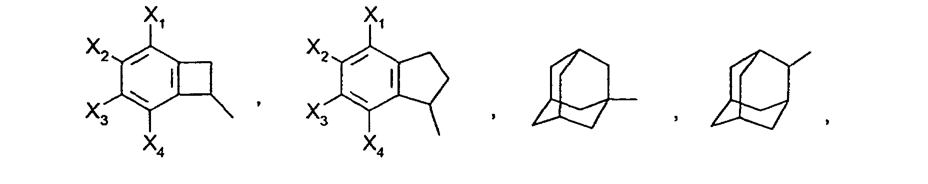 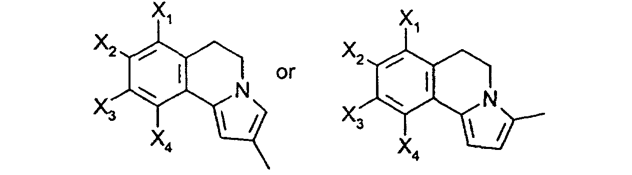
wherein :
. X₁, X₂, X₃ and X₄, which may be identical or different,
. each represents a hydrogen or halogen atom or (C₁-C₅)alkoxy radical in straight-chain or branched chain,
where they exist in the form of a racemic mixture or in the form of optical isomers,
and physiologically tolerable acid addition salts thereof

2. A compound of claim 1 selected from the group consisting of
N-[1-(2,3-dihydro[1,4]benzodioxin-5-yl)piperid-4-yl]-N-[2-(indan-1-yl)ethyl]amine and its hemi-fumarate,
N-[1-(2,3-dihydro[1,4]benzodioxin-5-yl)piperid-4-yl]-N-(indan-1-ylmethyl)amine and its fumarate,
N-[1-(2,3-dihydro[1,4]benzodioxin-5-yl)piperid-4-yl]-N-[3-(indan-1-yl)propyl]amine and its fumarate,
N-(benzocyclobuten-1-ylmethyl)-N-[1-(2,3-dihydro[1,4]benzodioxin-5-yl)piperid-4-yl]amine and its fumarate,
N-[3-(benzocyclobuten-1-yl)propyl]-N-[1-(2,3-dihydro[1,4]benzodioxin-5-yl)piperid-4-yl]amine and its fumarate,
N-[2-(benzocyclobuten-1-yl)ethyl]-N-[1-(2,3-dihydro[1,4]benzodioxin-5-yl)piperid-4-yl]-amine and its fumarate,
N-[4-(benzocyclobuten-1-yl)butyl]-N-[1-(2,3-dihydro[1,4]benzodioxin-5-yl)piperid-4-yl]-amine and its fumarate,
N-[1-(2,3-dihydro[1,4]benzodioxin-5-yl)piperid-4-yl]-N-(4,5-dimethoxybenzocyclobuten-1-ylmethyl)amine and its fumarate,
N-[2-(adamant-1-yl)ethyl]-N-[1-(2,3-dihydro[1,4]benzodioxin-5-yl)piperid-4-yl]amine and its fumarate,
N-[adamant-1-ylmethyl)-N-[1-(2,3-dihydro[1,4]benzodioxin-5-yl)piperid-4-yl]amine and its fumarate,
5,6-dihydro-8,9-dimethoxy-2-{2-{[1-(2,3-dihydro[1,4]benzodioxin-5-yl)piperid-4-ylamino]ethyl}}pyrrolo[2,1-a]isoquinoline and its hemi-fumarate,
5,6-dihydro-2-{2-{[1-(2,3-dihydro[1,4]benzodioxin-5-yl)piperid-4-ylamino]ethyl}}pyrrolo[2,1-a]isoquinoline and its hemi-fumarate,
5,6-dihydro-9-methoxy-2-{2-{[1-(2,3-dihydro[1,4]benzodioxin-5-yl)piperid-4-ylamino]ethyl}}pyrrolo[2,1-a]isoquinoline and its hemi-fumarate,
1-(2,3-dihydro[ 1,4]benzodioxin-5-yl)-4-{N-benzyl-2-(indan-1-yl)ethylamino}piperidine and its fumarate,
5,6-dihydro-8,9-dimethoxy-2-{[1-(2,3-dihydro[1,4]benzodioxin-5-yl)piperid-4-ylamino]methyl}pyrrolo[2,1-a]isoquinoline and its hemi-fumarate,
N-[2-(adamant-2-yl)ethyl]-N-[1-(2,3-dihydro[1,4]benzodioxin-5-yl)piperid-4-yl]amine and its fumarate,
N-[2-(adamant-1-yl)ethyl]-N-[1-(chroman-8-yl)piperid-4-yl]amine and its fumarate,
5,6-dihydro-8,9-dimethoxy-2-{2-[1-(chroman-8-yl)piperid-4-ylamino]ethyl}pyrrolo[2,1-a]isoquinoline and its hemi-fumarate, and
5,6-dihydro-8,9-dimethoxy-2-{2-[1-(6-fluorochroman-8-yl)piperid-4-ylamino]ethyl}pyrrolo[2,1-a]isoquinoline and its hemi-fumarate.

3. A compound according to claims 1 and 2 which is N-(benzocyclobuten-1-ylmethyl)-N-[1-(2,3-dihydro[1,4]benzodioxin-5-yl)piperid-4-yl]amine and its fumarate.

4. A compound according to claims 1 and 2 which is N-[2-(adamant-1-yl)ethyl]-N-[1-(2,3-dihydro[1,4]benzodioxin-5-yl)piperid-4-yl]amine and its fumarate.

5. A compound according to claims 1 and 2 which is 5,6-dihydro-8,9-dimethoxy-2-{2-{[1-(2,3-dihydro[ 1,4]benzodioxin-5-yl)piperid-4-ylamino]ethyl}}pyrrolo[2,1-a]isoquinoline and its hemi-fumarate.

6. A process for the preparation of compounds of claim 1, **characterised in that**
- a compound of formula II :
A-(CH₂)ₘ₋₁-CO₂H (II),
wherein A and m are as defined in claim 1, is condensed with an amine of formula III: 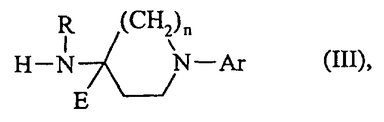
wherein R, E, n and Ar are as defined in claim 1,
by means of carbonyldiimidazole in dichloromethane, to obtain a compound of formula IV : 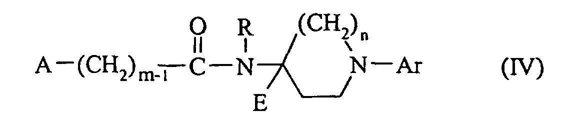 wherein A, m, R, E, n and Ar are as defined hereinbefore,
which is reduced to a compound of formula I by means of borane-dimethyl sulphide in tetrahydrofuran,
if desired, the isomers of compounds of formula I containing one or more asymmetric carbon atoms are prepared according to conventional methods known from the literature, and, optionally, the compounds I so obtained are treated with pharmaceutically acceptable acids to obtain the corresponding acid addition salts.

7. Pharmaceutical compositions, which can be used in the treatment of psychiatric disorders, degenerative diseases, pain, migraine, headaches, cerebral vascular accidents, bulimia, anorexia, drug abuse and cardiovascular disorders, comprising a compound according to any one of claims 1 to 5 together with one or more appropriate pharmaceutical excipients.
